# FASCICULE DE BREVET EUROPEEN

(11) **EP 0 501 848 B1**
(45) Date de publication et mention de la délivrance du brevet: **09.08.1995**
(21) Numéro de dépôt: 92400410.4
(22) Date de dépôt: 17.02.1992
(51) Int. Cl.: C07C 7/08

(54) **Procédé de séparation de butanes et de butènes par distillation extractive**
Verfahren zur Trennung von Butanen und Butenen durch extraktive Destillation
Process for the separation of butanes and butenes by extractive distillation

(30) Priorité: 26.02.1991 FR 9102385
(43) Date de publication de la demande: 02.09.1992
(73) Titulaire: INSTITUT FRANCAIS DU PETROLE, 92502 Rueil-Malmaison (FR)
(72) Inventeur: Asselineau, Lionel, F-75017 Paris (FR); Mikitenko, Paul, F-78590 Noisy Le Roi (FR)

(56) Documents cités:
- EP-A- 0 216 991

## Description

L'invention concerne un procédé de séparation des butanes et des butènes par distillation extractive.

Dans le traitement de la coupe C₄ issue du vapocraquage ou du craquage catalytique, la séparation des butènes-butanes se situe généralement en aval de la distillation extractive du butadiène-1,3 et de l'unité de synthèse du méthyl tertiobutyl éther (MTBE). On dispose donc d'une coupe C₄ débarrassée du butadiène-1,3 et très appauvrie en isobutène.

Les butènes étant des produits valorisables, on est amené à séparer les butènes des butanes. En effet, les butènes peuvent être soit eux-mêmes séparés en butène-1 et butènes-2, soit isomérisés en isobutène, qui est recyclé dans l'unité de synthèse de MTBE. Les butènes peuvent être également dimérisés par le procédé DIMERSOL (marque déposée) en produits utilisables dans les essences. Cette dimérisation est un procédé réalisé par catalyse homogène, dans lequel le catalyseur est perdu. L'efficacité du catalyseur étant proportionnelle à sa concentration dans la charge, une charge débarrassée de paraffines permet une économie de catalyseur. De plus, les butènes qui n'ont pas réagi peuvent être recyclés si la charge a été préalablement débarrassée des butanes.

Il est connu de séparer les butanes et les butènes dans une coupe C₄ par distillation extractive. Un procédé décrit antérieurement (S. OGURA, T. ONDA, Advances in C₄ Hydrocarbon Processing AIChE 1987 Summer National Meeting, August 16-19, 1987) consiste à réaliser la distillation extractive avec comme solvant le diméthylformamide (DMF) sous pression, la récupération du solvant étant effectuée sous pression atmosphérique et la purification des butènes étant effectuée sous pression. Le produit de fond de la colonne de distillation extractive est envoyé dans une colonne sous pression atmosphérique, dans laquelle les butènes sont désorbés et sortent en tête avec une partie du solvant, tandis que le produit de fond est constitué de DMF pratiquement pur. Les butènes de tête sont ensuite envoyés, pour être purifiés, dans une colonne sous pression. Le solvant récupéré en fond de cette dernière colonne et qui contient encore des butènes, retourne dans la colonne atmosphérique.

L'inconvénient d'opérer suivant ce procédé est que la majorité des butènes de la charge doit être distillée deux fois, ce qui, compte tenu de la recompression nécessaire des vapeurs de butènes, entraîne une consommation énergétique élevée.

On a maintenant découvert un nouveau procédé de séparation des butènes et des butanes par distillation extractive, qui présente l'avantage d'éviter la double distillation des butènes de la charge, et de ne recourir qu'à une recompression limitée des vapeurs de butènes.

Comme le procédé de l'art antérieur décrit plus haut, le procédé de l'invention comprend une étape de distillation extractive sous pression, mais les étapes de régénération du solvant et de purification des butènes sont effectuées de manière différente, comme cela apparaîtra dans la description qui suit.

On connait également le document EP-A-O 216 991 qui décrit un procédé de séparation de butanes et de butènes comportant une étape de distillation extractive des butènes par la morpholine anhydre et une étape de désorption totale des butènes. Est prévue en outre une étape facultative de purification (ou de régénération) du solvant exempt de butènes, destinée à séparer en fond les impuretés du solvant avant de le réintroduire dans le recyclage vers l'étape d'extraction.

Le procédé de séparation des butanes et butènes de l'invention peut être défini, d'une manière générale, en ce que la charge comprenant les butanes et les butènes à séparer est introduite dans une colonne de distillation extractive sous pression C1, où elle est mise en contact avec un solvant polaire dans lequel les butanes ont une volatilité plus élevée que les butènes, le distillat sortant en tête consistant essentiellement en les butanes séparés; le résidu recueilli en fond, comprenant principalement le solvant et les butènes, est envoyé dans une colonne de désorption sous pression C2 dans laquelle la température de fond est ajustée de manière qu'on réalise une désorption partielle desdits butènes, qui sortent en tête; le solvant qui sort en fond, contenant le reste des butènes, est envoyé dans une colonne de purification C3 sous une pression voisine de la pression atmosphérique, dont la marche est réglée de manière que l'effluent de tête contienne des butènes et une fraction du solvant, ledit effluent étant ensuite partiellement condensé, le condensat étant renvoyé en reflux dans ladite colonne du purification C3, et la phase vapeur étant renvoyée après compression dans ladite colonne de désorption sous pression C2; et le solvant purifié sortant en fond est recyclé vers la colonne de distillation extractive C1.

Il est important de noter que le fait d'ajuster la température de fond de la colonne de désorption C2 de manière que la désorption des butènes ne soit pas complète permet de imiter ladite température de fond à des hauteurs qui évitent la décomposition thermique du solvant, qui risque d'avoir lieu lorsqu'on met en oeuvre le procédé du document EP-A-0 216 991. Par ailleurs, le fait de régler la marche de la colonne de purification C3 de manière que la vapeur de tête de celle-ci, constituée principalement de butènes, contienne aussi une fraction du solvant, permet une condensation partielle de ladite vapeur, assurant par le condensat le reflux de ladite colonne, sans compression à ce stade, seul le distillat vapeur étant comprimé avant d'être recyclé vers la colonne de désorption sous pression C2.

La charge à traiter par le procédé de l'invention est en général une coupe C₄ de vapocraquage ou de craquage catalytique qui a été débarrassée du butadiène-1,3, par exemple par distillation extractive, et dont la teneur en isobutène peut avoir été réduite, par exemple dans une unité de synthèse du MTBE.

Les charges considérées comprennent donc en général principalement des butènes (butène-1, butène-2 cis et butène-2 trans), du n-butane, de l'isobutane, de l'isobutène en faible proportion et, à l'état de traces, des hydrocarbures à 3 et 5 atomes de carbone.

Une composition plus particulière de la charge peut être par exemple :

| | |
|---|---|
| Isobutane | de 15 à 20 % en poids |
| N-butane | de 7 à 15 % en poids |
| Butène-1 | de 20 à 25 % en poids |
| Isobutène | <5 % en poids |
| Butène-2 trans | de 25 à 30 % en poids |
| Butène-2-cis | de 15 à 20 % en poids |

Le solvant à utiliser dans le procédé de l'invention peut être tout solvant polaire sélectif, c'est-à-dire dans lequel les butanes ont une volatilité plus élevée que les butènes. A titre d'exemples non limitatifs, on peut citer le monométhylformamide, le diméthylformamide, le diéthylformamide, le diméthylacétamide et la N-méthylpyrrolidone. On utilise le plus souvent le diméthylformamide.

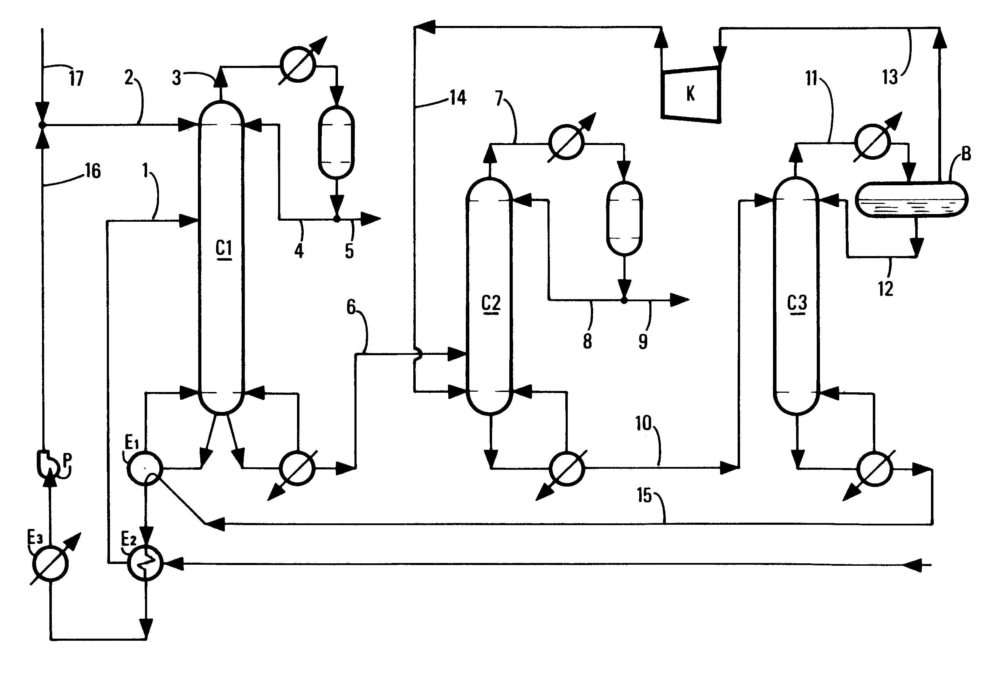

Le procédé de l'invention sera décrit ci-après de façon plus détaillée en liaison avec la figure annexée qui représente un agencement simplifié pour sa mise en oeuvre.

La charge à traiter, préalablement réchauffée à une température de 50 à 70 °C par exemple à travers l'échangeur E₂, est introduite par la ligne 1 dans la colonne de distillation extractive C₁ sous une pression de 4 à 10 bars.

Le solvant d'extraction, à une température inférieure à sa température d'ébullition à la pression considérée, est introduit, par la ligne 2, dans la partie supérieure de la colonne C₁. La température d'introduction du solvant est par exemple de 50 à 90 °C. Le débit de solvant peut être avec le débit de charge dans un rapport pondéral de 3 à 15 kg/kg.

La colonne C1 travaille par exemple avec une température de fond de 90 à 140 °C. La température de tête s'établit à une valeur de 30 à 70 °C.

Le distillat sortant en tête par la ligne 3 comprend principalement l'isobutane et le n-butane et, éventuellement, un peu de butène-1 et d'isobutène, en fonction du réglage de la colonne. Une fraction du distillat, après condensaton, est renvoyée, à titre de reflux vers la colonne C₁, par la ligne 4. On recueille le reste de l'effluent par la ligne 5, qui peut par exemple être renvoyé au pool C₄.

Le résidu de fond de colonne, très appauvri en butanes, est envoyé par la ligne 6 vers la partie inférieure de la colonne de désorption C₂ sous une pression voisine de celle de la colonne C₁. La température de fond de la colonne C₂, inférieure aux températures auxquelles le solvant commence à s'altérer, est réglée de manière à réaliser une désorption partielle des butènes. Elle est par exemple de 150 à 170 °C. La température de tête peut être de 30 à 60 °C.

L'effluent sortant en tête par la ligne 7 comprend essentiellement du butène-1 et des butènes-2, ainsi qu'éventuellement une faible proportion d'isobutène. Il est condensé et renvoyé en partie à titre de reflux vers la colonne C₂ par la ligne 8. On recueille le reste de l'effluent par la ligne 9.

Le résidu de fond de colonne C₂, sortant par la ligne 10, consiste en un flux de solvant contenant encore une fraction des butènes, par exemple de 3 à 5 % en poids. Il est envoyé vers la partie supérieure de la colonne de purification C₃ travaillant à une pression voisine de 1 bar (par exemple de 0,7 à 1,5 bars) et entre une température de fond de 140 à 170 °C et une température de tête de 120 à 150 °C.

L'effluent sortant en tête de la colonne C₃ par la ligne 11 est partiellement condensé.

La phase liquide est renvoyée à titre de reflux, du ballon de reflux B, vers la colonne C₃ par la ligne 12. La phase vapeur constituée essentiellement de butènes et contenant une faible proportion (par exemple de 3 à 6 % en poids) de solvant est envoyée par la ligne 13 vers un compresseur K, dans lequel elle est portée à la pression de la colonne C₂, avant d'être introduite par la ligne 14 dans la partie inférieure de la colonne C₂.

Le résidu de fond de colonne, sortant par la ligne 15 est du solvant pratiquement pur. Il est recyclé vers la colonne de distillation extractive C₁. L'énergie calorifique transportée par ce flux de solvant, en général à une température de 150 à 170 °C, peut être utilisée en partie pour chauffer le fond de la colonne C₁ par l'intermédiaire de l'échangeur E₁ et en partie pour réchauffer la charge jusqu'à son point de bulle par l'intermédiaire de l'échangeur E₂. Le flux de solvant peut encore être refroidi à travers l'échangeur E₃ avant d'être envoyé par la pompe P et la ligne 16 dans la colonne C₁. Un appoint de solvant peut être effectué par la ligne 17.

Par le procédé de l'invention, on peut obtenir les butènes avec un degré de pureté par exemple d'environ 97 % en poids ou davantage si nécessaire, principalement selon la proportion de solvant utilisé et l'efficacité (nombre de plateaux) de la colonne de distillation extractive.

Dans la présente description, les pressions indiquées sont des pressions absolues. On rappelle que le bar vaut 0,1 MPa.

On donne ci-après un exemple de réalisation pratique du procédé de l'invention.

EXEMPLE : la charge à traiter a la composition présentée dans la deuxième colonne du tableau.

Le solvant utilisé est le diméthylformamide anhydre, de pureté supérieure à 99,8 %.

Les colonnes utilisées présentent les caractéristiques suivantes :
- La colonne de distillation extractive C₁ est une colonne en acier de 50 mm de diamètre qui comporte 100 plateaux perforés à déversoir.
- La colonne de désorption C₂ est une colonne en acier de 50 mm de diamètre qui comporte 20 plateaux perforés à déversoir.
- La colonne de purification C₃ est une colonne en verre de 50 mm de diamètre que comporte 10 plateaux perforés à déversoir.

Les trois colonnes sont rendues adiabatiques, par un système de compensation de pertes thermiques pour les colonnes en acier et par une isolation thermique pour la colonne en verre.

On donne ci-après les conditions de fonctionnement des colonnes et les caractéristiques des flux de matière. Pour chaque colonne les plateaux sont comptés de haut en bas.

La colonne C₁ fonctionne sous une pression moyenne de 5,6 bars ; elle est alimentée au niveau du 62e plateau par la charge à 50 °C, sous un débit de 436 g/h, et par le solvant issu de la colonne C₃, amené à 65 °C. Le débit d'entrée du solvant au niveau du 10e plateau est de 2814 g/h. Un reflux de distillat représentant 930 g/h est envoyé en tête de colonne.

Le débit et la composition de l'effluent de tête après condensation (distillat) sont présentés dans la troisième colonne du tableau.

| | Colonne C1 | | Colonne C2 | |
|---|---|---|---|---|
| | Charge | Distillat | Distillat | Résidu |
| Débit g/h | 436 | 128 | 307 | 2928 |

| Composition % poids | | | | |
|---|---|---|---|---|
| isobutane | 18,37 | 62,18 | 0,02 | - |
| n-butane | 10,45 | 30,18 | 2,19 | 0,02 |
| butène-1 | 23,33 | 7,19 | 30,08 | 0,87 |
| isobutène | 1,54 | 0,38 | 2,03 | 0,06 |
| butène-2 trans | 27.91 | 0,06 | 39,57 | 1,59 |
| butène-2 cis | 18,40 | 0,01 | 26,11 | 1,16 |
| DMF | - | <0,01 | <0,01 | 96,30 |

La colonne C₂ fonctionne à une pression moyenne de 5,4 bars ; elle est alimentée au niveau du 18e plateau par le résidu précédent et au niveau du 20e plateau à 130 °C par 114 g/h de l'effluent vapeur de la colonne de purification C₃ (recyclage), préalablement comprimé à 5,5 bars. On établit un reflux de 130 g/h de distillat.

Le débit et la composition de l'effluent de tête après condensation (distillat) ainsi que ceux du résidu sont donnés respectivement en colonnes 4 et 5 du tableau.

La colonne C₃ fonctionne sous pression atmosphérique ; elle est alimentée au niveau du 1er plateau à 140 °C par le résidu de la colonne précédente. On établit un reflux liquide de 200 g/h, provenant de la condensation partielle de l'effluent de tête. La fraction vapeur de l'effluent de tête est recyclée à la colonne C₂, comme indiqué précédemment. En fond de colonne C₃ on recueille le DMF quasi pur, qui est retourné à la colonne C₁.

## Revendications

1. Procédé de séparation de butanes et de butènes à partir d'une charge en contenant, dans lequel ladite charge est introduite dans une colonne de distillation extractive sous pression C1, dans laquelle elle est mise en contact avec un solvant polaire dans lequel les butanes ont une volatilité plus élevée que les butènes, le distillat sortant en tête consistant essentiellement en les butanes séparés, ledit procédé étant caractérisé en ce que le résidu recueilli en fond, comprenant principalement le solvant et les butènes, est envoyé dans une colonne de désorption sous pression C2, dans laquelle la température de fond est ajustée de manière qu'on réalise une désorption partielle desdits butènes, qui sortent en tête ; le solvant qui sort en fond, contenant le reste des butènes, est envoyé dans une colonne de purification C3 sous une pression voisine de la pression atmosphérique, dont la marche est réglée de manière que l'effluent de tête contienne des butènes et une fraction du solvant, ledit effluent étant ensuite partiellement condensé, le condensat étant renvoyé en reflux dans ladite colonne de purification C3, et la phase vapeur étant renvoyée après compression dans ladite colonne de désorption sous pression C2; et le solvant purifié sortant en fond est recyclé vers la colonne de distillation extractive C1.

2. Procédé selon la revendication 1, caractérisé en ce que la charge à traiter est une coupe C4 de vapocraquage ou de craquage catalytique débarrassée du butadiène-1,3 et dont la teneur en isobutène a été réduite.

3. Procédé selon l'une des revendications 1 et 2, caractérisé en ce que la charge à traiter comprend principalement des butènes (butène-1, butène-2 cis et butène-2 trans), du n-butane, de l'isobutane, de l'isobutène en faible proportion et, à l'état de traces, des hydrocarbures à 3 et 5 atomes de carbone.

4. Procédé selon l'une des revendications 1 à 3, caractérisé en ce que ledit solvant polaire est choisi parmi le monométhylformamide, le diméthylformamide, le diéthylformamide, le diméthylacétamide et la N-méthylpyrrolidone.

5. Procédé selon l'une des revendications 1 à 4, caractérisé en ce que la colonne de distillation extractive C1 fonctionne à une pression de 4 à 10 bars ; le solvant est introduit dans la partie supérieure de ladite colonne de distillation extractive sous un débit dans un rapport pondéral de 3 à 15 avec le débit de ladite charge ; la température de fond de colonne est de 90 à 140 °C, et la température de tête de 30 à 70 °C.

6. Procédé selon l'une des revendications 1 à 5, caractérisé en ce que la pression de la colonne de désorption C2 est de 4 à 10 bars, la température de fond, inférieure à la température de décomposition du solvant, est de 150 à 170 °C, et la température de tête de 40 à 60 °C.

7. Procédé selon l'une des revendications 1 à 6, caractérisé en ce que la température de fond de ladite colonne de désorption C2 est ajustée de manière que son résidu de fond contienne de 3 à 5 % en poids de butènes.

8. Procédé selon l'une des revendications 1 à 7, caractérisé en ce que la pression de la colonne de purification C3 est d'environ 1 bar, la température de fond est de 140 à 170 °C et la température de tête de 120 à 150 °C.

9. Procédé selon l'une des revendications 1 à 8, caractérisé en ce que les conditions de marche de ladite colonne de purification C3 sont telles que l'effluent de tête de ladite colonne comprenne de 3 à 6 % en poids de solvant.

10. Procédé selon l'une des revendications 1 à 9, caractérisé en ce que les conditions de marche du procédé sont telles que l'on obtient en tête de la colonne de désorption C2 un mélange de butènes ayant une pureté d'au moins 97 % en poids.

## Claims

1. Process for the separation of butanes and butenes from a charge containing the same, in which the said charge is introduced into an extractive distillation column under pressure C₁, in which it is contacted with a polar solvent, wherein the butanes have a higher volatility than the butenes, the distillate leaving at the head consisting essentially of the butanes separated, said process being characterized in that the residue collected at the bottom, and comprising mainly the solvent and the butenes is fed into a desorption column under pressure C₂, in which the bottom temperature is controlled so as to achieve a partial desorption of said butenes, which leave at the head; the solvent leaving at the bottom and which contains the remaining butenes is fed into a purification column C₃ under a pressure close to atmospheric pressure and the operation of which is controlled so that the head effluent contains butenes and a fraction of said effluent is then partly condensed, the condensate is returned as reflux to the purification column C₃ and the vapour phase is returned after compression into the pressure desorption column C₂; and the purified solvent leaving at the bottom is recycled to the extractive distillation column C₁.

2. Process according to claim 1, characterized in that the charge to be treated is a C₄-cut from steam-cracking or catalytic cracking free from 1,3-butadiene and whose content of isobutene has been reduced.

3. Process according to anyone of the claims 1 and 2, characterized in that the charge to be treated mainly comprises butenes (1-butene, cis 2-butene and trans 2-butene), n-butane, isobutane, small proportions of isobutene and trace hydrocarbons having 3 or 5 carbon atoms.

4. Process according to anyone of the claims 1 to 3, characterized in that the polar solvent is chosen from among monomethyl formamide, dimethyl formamide, diethyl formamide, dimethyl acetamide and methyl pyrrolidone.

5. Process according to anyone of the claims 1 to 4, characterized in that the extractive distillation column C₁ operates at a pressure of 4 to 10 bars; the solvent is introduced into the upper part of said extractive distillation column with a flow which is in a weight ratio of 3 to 15 with the flow of said charge; and the column bottom temperature is 90 to 140°C and the head temperature 30 to 70°C.

6. Process according to anyone of the claims 1 to 5, characterized in that the pressure in the desorption column C₂ is 4 to 10 bars, the bottom temperature, which is below the decomposition temperature of the solvent, is 150 to 170°C and the head temperature is 40 to 60°C.

7. Process according to anyone of the claims 1 to 6, characterized in that the bottom temperature of said desorption column C₂ is controlled so that its bottom residue contains 3 to 5% by weight butenes.

8. Process according to anyone of the claims 1 to 7, characterized in that the pressure of the purification column C₃ is approximately 1 bar, the bottom temperature is 140 to 170°C and the head temperature 120 to 150°C.

9. Process according to anyone of the claims 1 to 8 characterized in that the operating conditions of said purification column C₃ are such that the head effluent of said column comprises 3 to 6% by weight solvent.

10. Process according to anyone of the claims 1 to 9, characterized in that the operating conditions of the process are such that a mixture of butenes having a purity of at least 97% by weight is obtained at the head of the desorption column C₂.

## Patentansprüche

1. Verfahren zum Trennen von Butanen und Butenen aus einer sie enthaltenden Charge, bei dem diese Charge in eine unter Druck stehende Extraktivdestillationskolonne C1 eingeführt wird, in der sie mit einem polaren Lösungsmittel kontektiert wird, in welchem die Butane eine höhere Flüchtigkeit als die Butene haben, wobei das am Kopf austretende Destillat im wesentlichen aus den abgetrennten Butanen besteht, wobei das Verfahren sich dadurch auszeichnet, daß der am Boden gesammelte Rückstand, der hauptsächlich das Lösungsmittel und die Butene umfaßt, in eine unter Druck stehende Desorptionskolonne C2 geschickt wird, in der die Bodentemperatur derart eingestellt wird, daß man eine teilweise Desorption dieser Butene, die am Kopf austreten, durchführt; daß das den Rest der Butene enthaltende Lösungsmittel, daß am Boden austritt, in eine Reinigungskolonne C3 geschickt wird, die unter einem Druck benachbart Atmosphärendruck steht, deren Betrieb derart eingestellt wird, daß dar Kopfabstrom Butene und eine Fraktion des Lösungsmittels enthält, wobei der Abstrom dann teilweise kondensiert, das Kondensat im Rückstrom in diese Reinigungskolonne C3 rückgeschickt und die Dampfphase nach Kompression in diese unter Druck stehende Desorptionskolonne C2 zurückgeschickt wird; und das gereinigte am Boden austretende Lösungsmittel zur Extraktivdestillationskolonne C1 recycliert wird.

2. Verfahren nach Anspruch 1, dadurch gekennzeichnet, daß die zu behandelnde Charge eine C4-Fraktion aus der Dampfcrackung oder katalytischen Crackung ist, die vom 1,3-Butadien befreit wurde und deren Gehalt an Isobuten reduziert worden ist.

3. Verfahren nach einem der Ansprüche 1 und 2, dadurch gekennzeichnet, daß die zu behandelnde Charge hauptsächlich Butene (1-Buten, cis-2-Buten und trans-2-Buten), n-Butan, Isobutan, Isobuten in geringem Anteil und im Zustand von Spuren, Kohlenwasserstoffe mit 3 bis 5 Kohlenstoffatomen umfaßt.

4. Verfahren nach einem der Ansprüche 1 bis 3, dadurch gekennzeichnet, daß dieses polare Lösungsmittel gewählt ist aus Monomethylformamid, Dimethylformamid, Diethylformamid, Dimethylacetamid und N-Methylpyrrolidon.

5. Verfahren nach einem der Ansprüche 1 bis 4, dadurch gekennzeichnet, daß die Extaktivdestillationskolonne C1 unter einem Druck von 4 bis 10 bar arbeitet; das Lösungsmittel in den oberen Teil dieser Extraktivdestillationskolonne mit einem Durchsatz in einem Gewichtsverhältnis von 3 bis 15 zum Durchsatz dieser Charge eingeführt wird; die Kolonnenbodentemperatur 90 bis 140 °C und die Kopftemperatur 30 bis 70 °C beträgt.

6. Verfahren nach einem der Ansprüche 1 bis 5, dadurch gekennzeichnet, daß der Druck der Desorptionskolonne C2 4 bis 10 bar, die Bodentemperatur, die kleiner als die Zersetzungstemperatur des Lösungsmittels ist, 150 bis 170 °C und die Kopftemperatur 40 bis 60 °C beträgt.

7. Verfahren nach einem der Ansprüche 1 bis 6, dadurch gekennzeichnet, daß die Bodentemperatur dieser Desorptionskolonne C2 derart eingestellt wird, daß ihr Bodenrückstand 3 bis 5 Gew.-% Butene enthält.

8. Verfahren nach einem der Ansprüche 1 bis 7, dadurch gekennzeichnet, daß der Druck der Reinigungskolonne C3 etwa 1 bar, die Bodentemperatur 140 bis 170 °C und die Kopftemperatur 120 bis 150 °C beträgt.

9. Verfahren nach einem der Ansprüche 1 bis 8, dadurch gekennzeichnet, daß die Betriebsbedingungen der Reinigungskolonne C3 derart sind, daß der Kopfabstrom dieser Kolonne 3 bis 6 Gew.-% Lösungsmittel umfaßt.

10. Verfahren nach einem der Ansprüche 1 bis 9, dadurch gekennzeichnet, daß die Betriebsbedingungen des Verfahrens derart sind, daß man Kopf der Desorptionskolonne C2 ein Gemisch aus Butenen mit einer Reinheit von wenigstens 97 Gew.-% erhält.
